# EUROPEAN PATENT APPLICATION

(11) **EP 0 730 870 A1**
(43) Date of publication of application: **11.09.1996**
(21) Application number: 95401300.9
(22) Date of filing: 02.06.1995
(51) Int. Cl.: A61K 51/06, A61K 51/12

(54) **Radioactive chitosan complex and its macroaggregates for use in internal radiation therapy and their preparation method**

(30) Priority: 10.03.1995 KR 9504872
(71) Applicant: KOREA ATOMIC ENERGY RESEARCH INSTITUTE, Daejeon-si (KR)
(72) Inventor: Park, Kyoung-Bae, Yongsan-Ku, Seoul (KR); Kim, Young-Mi, Nowon-Ku, Seoul (KR); Kim, Jae-Rok, Sungdong-ku, Seoul (KR)
(74) Representative: Hirsch, Marc-Roger

(57) **Abstract**

Radioactive chitosan complex and its macroaggregate and method for preparing thereof is capable of substituting conventional surgical operation and medicinal treatment method for hepatocellular carcinoma or metastasized liver cancer. Chitosan complex and its macroaggregate, a natural high molecular substance having a non-toxicity, bio-compatability and biodegradability are labelled with radionuclide (¹⁵⁹Sm, ¹⁶⁵Dy, ¹⁶⁶Ho, ¹⁶⁹Er) of emitting high energy beta-rays. The radioactive chitosan macroaggregate prepared as above is intrapercutaneously or intraartery injected to lesion of liver, so that it can be selectively more accumulated to a cancer cell than a normal tissue, and can treat by selectively killing the cancer cell by beta-rays emitted from the said radioactive nuclides. An ovarium cancer, a stomach cancer, a peritoneol cavity cancer, and a rheumatoid arthritis which are all known as incurable diseases can also be treated by selectively killing its lesion by means of this internal radiation therapy.

## Description

### BACKGROUND OF THE INVENTION

The present invention relates to a radioactive chitosan and macroaggregates for internal radiation therapy and method for preparing thereof.

A treatment method of hepatocellular carcinoma or metastasized liver cancer can be largely classified to a surgical operation and medicinal treatment. Among the medicinal treatment method, in case of dosing an anti-cancer drug through an oral administration or intravenous injection, since the medicine is once spread to whole body through a blood flow and a little amount is even so accumulated to lesion in most case, therefore much quantity has to be dosed, and an adverse effect due to this is raised as a serious problem. In order to correct and compensate this disadvantage, it is a real situation that a treatment method of disrupting a cancer tissue by direct administration of anhydrous ethanol to liver cancer lesion by percutaneous injection is prevailed in Japan.

As another method, there is a method in which an anti-cancer drug is directly injected through a hepatic artery instead of intravenous injection so that relatively much quantity is accumulated to a cancer tissue whereby increasing a curing effect.. In order to increase a selectivity, an anti-cancer drug is emulsified with oil such as lipiodol which has high viscosity so that sustained-release of drug is intended. Although a method was also introduced in which radioactive ¹³¹I is directly labelled to the lipiodol whereby curing by beta-rays emitted therefrom, since gamma-rays of relatively high energy is also emitted simultaneously, radiation damage to other organ or tissue can also become a problem and a quantity accumulated to lungs after hepatic artery injection in some case is also much, it could not be generalized.

Recently, active research is carried out in which fine particles (15-50µm) labelled with radionuclide are directly injected to intra-hepatic artery whereby capillary blood vessel within a cancer tissue is embolized and not only nourishment supply is stopped but also beta-rays are emitted at the same time whereby double curing effect is intended. As particles capable of carrying the radionuclide, those which consisted of non-degradable substance such as resin [Turner et al., Nucl. Med. Comm. 15, 545 (1994)], ceramic, glass [Andrews et al., J. Nucl. Med., 35, 1637 (1994)] and the like are known substances, and also there was a report related to a radioactive macroaggregates labelled with ¹⁶⁶Ho to poly-L-latic acid which is an artificial biodegradable polymer [(Russell et al., J. Nucl. Med. 33, 398 (1992)].

In case of using the radioactive particles care should be taken that the radionuclides not be isolated from the particles so as not to be accumulated through a blood flow to other organs or tissues particularly to bone marrows. For this, non-degradable substance has been mainly used, but it may be further ideal if it is a biodegradable and a bio-compatible substance in which radioactive decay is almost completed and thereafter particles are naturally decomposed in tissue resulting stable nuclear species and decomposed substance and subsequently discharged to exterior of body.

In treatment of rheumatoid arthritis, especially case when the arthritis is chronically continued inspite of contineous medicinal treatment, a surgical synovectomy or radiation synovectomy is applied. The radiation synovectomy is a plausible technique of replacing surgical synovectomy, and recently, active research has been carried out toward its clinical application, and it is a method capable of easy removal of inflammed region of synovium by beta-radiation through the direct injection with small amounts of particles labelled with β emitting radionuclide.

Those presently used are ¹⁶⁶Dy-FHMA [(Harling et al., Nucl. Sci. and Eng., 110, 344 (1992)], ¹⁶⁵Dy-HMA [McLaren et al., Eur. J. Nucl. Med., 16, 627 (1990)], ⁹⁰Y-silicate, etc. Recently, there are reports on ¹⁵³Sm-hydroxyapatite which is an inorganic component having biodegradability and bio-compatibility [Chinol et al., J. Nucl. Med., 34, 1536 (1993)], silicate bead of alkali metal ion [USP 5011797], glass beads contained with ¹⁵³Sm, ¹⁶⁵Dy, ¹⁶⁶Ho, ⁹⁰Y etc, and an organic component, ¹⁶⁶Ho-poly-L-lactic acid.

In the present invention, chitosan is used as a carrier of radionuclids. Chitosan is abundant in nature, biodegradable and bio-compatible. It is not a non-degradable particle of inorganic substance as previously refered as a carrier of radionuclides and which is also not an artificial biodegradable organic substance as poly-L-lactic acid. The chitosan can easily be obtained by alkaline hydrolysis of chitin being abundant next to cellulose in nature, and chitosan is much contained particularly in shells of shirimp, lobster, crab and oyster and they have been left alone as a waste matter in food industry, but recently a research for these has become active, aiming resources recycling.

The chitosan is known as an edible non-toxic substance as a food preserving agent in food industry, particularly as a heavy metal disposing agent, and various properties have been reported as a biodegradable stiching yarn, an artificial kidney membrane, a skin protecting membrane upon burn curing, and anti-acidic, anti-gastric ulcer, and anti-cancer agent in medical and medicinal industry, and recently it is also widely known as an applying agent for sustained release of drugs, and thereby a remarkable concern in pharmaceutical business [Kubota et al., Chem. Soc. Jpn., 66, 1807 (1993)].

### DETAILED DESCRIPTION OF THE INVENTION

In considering above mentioned properties of chitosan, we have tried to label it with radionuclides of the lanthanides such as ¹⁵³Sm, ¹⁶⁵Dy, ¹⁶⁶Ho, ¹⁶⁹Er which are mostly strong β⁻ but weak γ-emitters. We thereby have succeeded eventually to obtain noble radioactive chitosan complex in solution. In considering their in-vivo and in-vitro stability, it is thought that a curing efficacy can be maximized by directly injecting it to lesion of incurable diseases such as liver cancer and rheumatoid arthritis and the like.

Details of the present invention are enumerated in the following claims and examples.

### EXAMPLE 1

### ¹⁶⁶Preparing of ¹⁶⁵Ho-chitosan Complex

Thirty five mg of chitosan (about 500,000 in molecular weight, hydrolysis rate 85%) was dissolved 4 ml of aqueous 2% acetic acid solution and then the pH was adjusted to 3.0. By neutron irradiation in nuclear reactor, it was converted to ¹⁶⁶Ho(NO₃)₃ and dissolved in 2 ml distilled water. 0.1 ml aliquot of the solution was added to chitosan solution, mixed well, and maintained at room temperature for thirty(30) minutes.

### EXAMPLE 2

### Examination for yield of ¹⁶⁶Ho-chitosan complex and its radiochemical purity

The resultant reaction mixture of example 1 was subjected to a instant thin layer chromatography(ITLC). Silicic acid coated glass fiber plate and the solvent mixture made of methanol:water:acetic acid (49:49:2 V/V) were, respectively, used as a stationary phase and mobile phase. The reaction yield was appeared over 99%. And, the radiochemical purity of the mixture was consecutively confirmed by ITLC at every time interval with maintaining the sample at room temperature or 37°C for inspection of its in-vitro stability. Rf values of ¹⁶⁶Ho-chitosan complex and the isolated ¹⁶⁶Ho were, respectively, appeared to be 0.3 and 0.9, and hence its resolution was excellent.

### EXAMPLE 3

### Examination of in-vitro stability of ¹⁶⁶Ho-chitosan complex

Radiochemical purity was examined for the ¹⁶⁶Ho-chitosan complex prepared in example 1 by ITLC as described in example 2 with maintaining at room temperature or 37°C and thereby the amount of ¹⁶⁶Ho isolated from the complex was shown by percentage. As shown in Table 1, the isolated ¹⁶⁶Ho was almost negligible even maintained for twenty-five(25) days at room temperature or 37°C, and hence it was confirmed that the in-vitro stability of ¹⁶⁶Ho-chitosan complex was very high.

**Table 1**

| The effect of time on the stability of ¹⁶⁶Ho-chitosan complex with time | | |
|---|---|---|
| Elapsed time (days) | Radiochemical purity of (%) | ¹⁶⁶Ho-chitosan complex |
| | Room temperature | 37°C |
| 0 | 94 | 99 |
| 2 | 99 | 99 |
| 4 | 99 | 99 |
| 6 | 99 | 99 |
| 15 | 99 | 99 |
| 25 | 99 | 99 |

### EXAMPLE 4

### Preparation of ¹⁵³Sm-¹⁶⁵Dy-¹⁶⁹Er-chitosan complexes and examination of their in-vitro stability

The complexes were prepared in accordance with the procedure described in example 1 and 2, and the in-vitro stability of the resultant complexes were examined. The reaction yield of these complex was all over 99%, and the in-vitro stability was as high as those of the ¹⁶⁶Ho case.

### EXAMPLE 5

### Preparation of ¹⁶⁶Ho-chitosan macroaggregates and their paticle size distribution

2N NaOH aqueous solution was slowly added to ¹⁶⁶Ho-chitosan complex solution prepared in example 1 with vigorous stirring. Alter completion of precipitate formation of precipitate was separated from the supernatant by centrifugation. Radioactivity of each part was measured. The radioactivity of the supernatant was negligible. Thus, we could confirm that the macroaggregate was formed almost quantitatively. The diameter of the particles were ranged in 10-40 µm region, and the average particle size was about 25 µm.

### EXAMPLE 6

### Examination of in-vitro stability of ¹⁶⁶Ho-chitosan Macroaggregates.

The macroaggregates prepared in example 5 was centrifugally separated and then washed six(6) times using 5 ml distilled water or physiological saline solution. At each washing, the radioactivities of precipitate and supernatant were measured. The radioactivity of supernatant was almost negligible indicating that the macroaggregate is quite stable.

**Table 2**

| Measurement of stability of 166Ho-chitosan macroaggregate by consecutive washing | | |
|---|---|---|
| Washing volume (ml) | Radioactivity of ¹⁶⁶Ho-chitosan macroaggregate | Radioactivity in supernatant (%) |
| 0 | 100 | 0 |
| 5 | 100 | 0 |
| 10 | 100 | 0 |
| 15 | 100 | 0 |
| 20 | 100 | 0 |
| 25 | 100 | 0 |
| 30 | 100 | 0 |

### EXAMPLE 7

### Examination of in-vitro stability of ¹⁶⁶Ho-chitosan macroaggregate

The macroaggregate prepared in example 5 was centrifugally separated at every time interval maintaining at room temperature or 37°C. The radioactivities of the supernatant and the precipitate were counted. The results indicated that the in-vitro stability of the ¹⁶⁶Ho-chitosan macroaggregate was excellent in case of both at room temperature and 37°C.

**Table 3**

| Effect of elapsed time on the stability of ¹⁶⁶Ho-chitosan macroaggregates | | |
|---|---|---|
| Elapsed time (days) | Radioactivity of macroaggregate | ¹⁶⁶Ho-chitosan (%) |
| | Room temperature | 37°C |
| 0 | 100 | 100 |
| 2 | 100 | 100 |
| 4 | 100 | 100 |
| 6 | 100 | 100 |
| 13 | 100 | 100 |
| 25 | 100 | 100 |

### EXAMPLE 8

### Examination of in-vivo stability of ¹⁶⁶Ho-chitosan complex and its macroaggregate

The pH of the complex solution prepared in example 1 was adjusted to 5.5 and then filtered through 0.2µm membrane filter and sterilized. 0.1 ml (0.5 mCi) portion was administered into knee joint of normal rabbit by intra articular injection.

After an appropriate time interval, radioactivity remained within the knee joint was compared with the total radioactivity immediately after the injection by using γ -camera. The results showed that the radioactivity was almost quanititatively remained in the joint cavity even upto 48 hours after injection (excluding physical decrease of ¹⁶⁶Ho) indicating that the prepared complex has sufficient in-vivo stability.

The macroaggregate was prepared according to example 5, suspended to saline solution, and thereafter sterilized by autoclaving at 121°C for 30 minutes. 0.1 ml (0.5 mCi) aliquot of this suspension was administered into the knee joint as described above. The results indicated that the in-vivo stability was as good as the complex.

**Table 4**

| Percentage of radioactivity of ¹⁶⁶Ho-chitosan macroaggregate remained within a rabbit articulation | | |
|---|---|---|
| Time (hour) | Radioactivity remained in articulate | |
| | ¹⁶⁶Ho-chitosan complex | ¹⁶⁶Ho-chitosan macroaggregate |
| 0 | 100 | 100 |
| 2 | 99.9 | 99.9 |
| 6 | 99.8 | 99.8 |
| 24 | 99.7 | 99.8 |
| 48 | 99.6 | 99.7 |

## Claims

1. An internal radiation therapeutic agent of radioactive chitosan complex prepared by tagging with radioactive nuclides.

2. The internal radiation therapeutic agent of claim 1, wherein the radioactive nuclides are lanthanides.

3. The internal radiation therapeutic agent of claim 2, wherein said radioactive lanthanides are selected from the group consisting of : ¹⁵³Sm, ¹⁶⁵Dy, ¹⁶⁶Ho and ¹⁶⁹Er.

4. An internal radiation therapeutic agent of radioactive chitosan macroaggregate prepared from chitosan complex according to claim 1, 2 or 3.

5. A method for preparing a internal radiation therapeutic agent according to claim 1, which comprises the steps of dissolution of chitosan into an aqueous acidic solution; providing a radioactive nuclide, under appropriate salt form and dissolution of same in aqueous solution; admixing both aqueous solutions; and optionally storing at room temperature.

6. A method for preparing a internal radiation therapeutic agent according to claim 4, which comprises the steps of the method of claim 4, followed by a step of adding a basic compound to form aggregates.

7. The method of claim 5 or 6, wherein the pH of the acidic solution is about 3, the radioactive nuclide salt is the (NO₃)₃ salt.
